(19) Europäisches Patentamt
European Patent Office
Office européen des brevets

(11) Numéro de publication: **0 065 446 B1**

(12) # FASCICULE DE BREVET EUROPEEN

(45) Date de publication du fascicule du brevet: **12.09.84**

(51) Int. Cl.³: **C 07 D 307/16,** A 61 K 31/34

(21) Numéro de dépôt: **82400799.1**

(22) Date de dépôt: **30.04.82**

(54) **Citrate de naftidrofuryl et son application thérapeutique.**

(30) Priorité: **14.05.81 FR 8109590**

(43) Date de publication de la demande:
**24.11.82 Bulletin 82/47**

(45) Mention de la délivrance du brevet:
**12.09.84 Bulletin 84/37**

(84) Etats contractants désignés:
**AT BE CH DE FR GB IT LI LU NL SE**

(56) Documents cités:
**FR - M - 3 843**

(73) Titulaire: **SANOFI, société anonyme, 40, Avenue George V, F-75008 Paris (FR)**

(72) Inventeur: **Blasioli, Carlo, Résidence Riva Bella 176 Avenue de Muret, F-31100 Toulouse (FR)**
Inventeur: **Chick, Jacques Alexandre, 2 Rue des Fontaines, F-92310 Sevres (FR)**
Inventeur: **Heymes, Alain, Chemin de l'Adrech, F-04200 Sisteron (FR)**

(74) Mandataire: **Lavoix, Jean et al, c/o Cabinet Lavoix 2, Place D'Estienne D'Orves, F-75441 Paris Cedex 09 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 065 446 B1

ACTORUM AG

## Description

La présente invention est relative à un nouveau sel de naftidrofuryl, à savoir le citrate de naftidrofuryl, qui possède des propriétés toxicologiques, pharmacologiques et cliniques intéressantes et bénéfiques pour son utilisation en thérapeutique.

Le brevet FR-M-3843 décrit de façon générale des sels d'acides de naftidrofuryl et mentionne spécifiquement le phosphate, le fumarate et l'oxalate de naphtidrofuryl, ce dernier étant en particulier utilisé en thérapeutique humaine pour ses propriétés d'activateur métabolique et circulatoire. Il est également doué de propriétés vasoactives par ses effets spasmolytique et sympatolytique.

Le naftidrofuryl ou (naphtyl-1)-3 tétrahydrofurfuryl-2 propionate de diéthylamino-2 éthyle répond à la formule développée suivante:

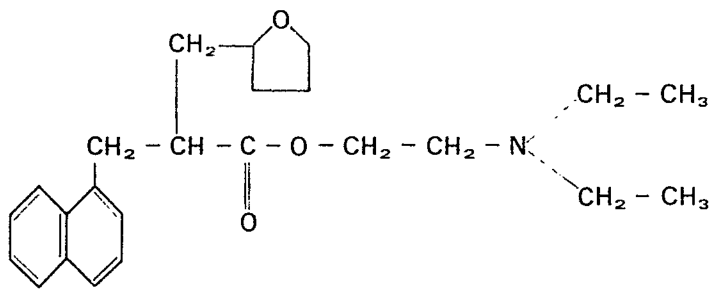

La présente invention a ainsi pour objet un sel particulier de naftidrofuryl présentant des propriétés améliorées, à savoir le citrate de naftidrofuryl et son application en thérapeutique.

On décrira ci-après, à titre d'exemple non limitatif, un procédé de fabrication du citrate de naftidrofuryl. Une étude comparative de l'oxalate et du citrate est également présentée, notamment en ce qui concerne la détermination de la $DL_{50}$ chez la souris et le rat, les effets cardiorespiratoires après perfusion chez le chien et la biodisponibilité réalisée chez l'homme.

Procédé de fabrication du citrate de naftidrofuryl:

Le citrate de naftidrofuryl est préparé par un procédé classique de salification à partir de la base libre en utilisant de l'acide citrique monohydraté suivant le mode opératoire décrit ci-après.

On mélange 2,5 l d'acétone, 2,5 l d'acétate d'éthyle, 500 g (1,30 mole) de naftidrofuryl base purifié et 2,74 g (1,30 mole) d'acide citrique monohydraté, puis on porte à reflux et refroidit vers 5°C. Le précipité est filtré et séché.

M: 640 g (Rdt: 85%)

P.F.: env. 84°C

Ce composé a été recristallisé dans 4 l d'un mélange acétone/acétate d'éthyle dans les proportions 1/1 (v:v).

M: 590 g (Rdt recristallisé: 92%)

P.F.: env. 86°C

*Analyse* pour ($C_{24}H_{33}NO_3$, $C_6H_8O_7$)

| | | | |
|---|---|---|---|
| Calculé: | C 62,59 | H 7,18 | N 2,43% |
| Trouvé: | C 62,01 | H 7,13 | N 2,48% |

Les résultats des études toxicologiques, pharmacologiques et cliniques qui sont rapportés ci-après mettent en évidence les intéressantes propriétés du dérivé de l'invention.

I — *Etude toxicologique*

Les essais ont été réalisés sur deux espèces animales, la souris et le rat, et par deux voies d'administration, tubage gastrique et voie endoveineuse.

I.1 — *Essais sur la souris*

Chaque dose essayée a été administrée sur des lots de 10 souris femelles de souche Swiss de poids corporel compris entre 22 et 25 g. Les deux sels de naftidrofuryl ont été administrés, pour la voie orale, en suspension dans une solution aqueuse de gomme arabique à 5% et sous un volume de 20 ml/kg et pour la voie intraveineuse dans une solution aqueuse de chlorure de sodium à 9‰ et sous un volume de 10 ml/kg. La $DL_{50}$ a été calculée selon la méthode par probits/ ordinateur.

I.1.1 — Administration par tubage gastrique: la $DL_{50}$/24 h/kg a été de 668,40 mg pour l'oxalate de naftidrofuryl et de 1224,79 mg pour le citrate de naftidrofuryl.

I.1.2 — Administration par voie intraveineuse: la $DL_{50}$/24 h/kg a été de 18,40 mg pour l'oxalate de naftidrofuryl et de 22,28 mg pour le citrate de naftidrofuryl.

I.2 — *Essais sur le rat*

La technique utilisée a été la même que celle précédemment décrite pour la souris. Les rats mâles de souche Wistar avaient un poids corporel compris entre 180 et 210 g. Le volume de l'administration pour la voie orale a été de 15 ml/kg et pour la voie injectable de 2 ml/kg.

I.2.1 — Administration par tubage gastrique: la $DL_{50}$/24 h/kg a été de 2293,44 mg d'oxalate de naftidrofuryl et de 3394,44 mg pour le citrate de naftidrofuryl.

I.2.2 — Administration par voie intraveineuse: la $DL_{50}$/24 h/kg a été de 11,08 mg pour l'oxalate de naftidrofuryl et de 13,56 mg pour le citrate de naftidrofuryl.

Le simple examen de l'ensemble de ces résultats montre une nette différence, la meilleure tolérance étant en faveur du citrate de naftidrofuryl. C'est ainsi que, par exemple, la $DL_{50}$/24 h/kg p.o. est supérieure de 83,2% chez la souris et de 48% chez le rat par rapport à l'oxalate de naftidrofuryl.

II — *Etude pharmacologique*

Les essais cardiorespiratoires après perfusion

ont été réalisés sur 10 chiens mâles de race Beagle pesant entre 13 et 16 kg.

Les animaux ont été placés sous perfusion soit d'oxalate de naftidrofuryl, soit de citrate de naftidrofuryl, à la concentration de 1,5 mg/ kg·$mn^{-1}$ sous 5 ml/mn, jusqu'à la mort de l'animal. les produits testés avaient été préalablement dissous dans un soluté aqueux de chlorure de sodium à 9‰.

II.1 – *Protocole expérimental*

Après anesthésie au nembutal et chloralose, la pression artérielle est relevée au niveau des artères carotide et fémorale par l'intermédiaire de cellules de pression de type Statham.

L'électrocardiogramme est enregistré selon la dérivation standard DII et permet de calculer la durée de l'espace PR et la fréquence cardiaque après traitement par un biotachomètre de type Narco.

La pression veineuse est contrôlée au niveau de la veine jugulaire par une cellule de pression de type Statham.

Les variations d'amplitude de la force de contraction cardiaque sont mesurées par une jauge de contrainte cousue sur le ventricule gauche parallèlement au sillon interventriculaire.

Trois débits sanguins sont relevés par la même méthode de débitmétrie électromagnétique:

– débit artériel fémoral et carotidien,
– débit cardiaque au niveau de l'aorte thoracique ascendante. De ce paramètre seront calculés le travail cardiaque, le volume d'éjection systolique et les résistances périphériques totales.

Une sonde thermocouple placée à la sortie d'une canule trachéale permet de déterminer la fréquence et l'amplitude respiratoire.

Tous ces paramètres sont enregistrés simultanément sur un polygraphe Beckman.

II.2 – *Résultats*

Dans les conditions expérimentales décrites, qui ont permis de déterminer les doses minimales mortelles des sels de naftidrofuryl.

II.2.1 – L'arrêt respiratoire a été obtenu à $28 \pm 5$ mg/kg avec l'oxalate de naftidrofuryl et à $41 \pm 4$ mg/kg avec le citrate de naftidrofuryl.

II.2.2 – Le collapsus cardiovasculaire qui a suivi est intervenu aux doses de $33 \pm 4$ mg/kg avec l'oxalate de naftidrofuryl et de $45 \pm 3$ mg/kg avec le citrate de naftidrofuryl.

Il apparaît ainsi une différence nette en faveur du citrate de naftidrofuryl pour obtenir arrêt respiratoire et collapsus cardiaque. Cet essai est donc en faveur d'une meilleure tolérance et d'une meilleure activité cardiaque et respiratoire du citrate de naftidrofuryl par rapport à l'oxalate de naftidrofuryl, les paramètres cardiovasculaires mesurés au cours de perfusions évoluant, eux aussi, dans le même sens.

Il y a lieu de noter que, pour lever l'hypothèque d'une éventuelle action propre à la partie oxalate ou citrate des molécules testées, des essais de contrôle ont été réalisés sur des chiens qui ont reçu, dans les mêmes conditions expérimentales précédemment décrites, soit de l'oxalate de sodium, soit du citrate de sodium: la perfusion a été poursuivie pendant 50 min sans que les paramètres observés aient présenté la moindre variation, alors que pas un seul chien ayant reçu soit l'oxalate de naftidrofuryl, soit le citrate de naftidrofuryl n'a résisté plus de 25 min à la perfusion.

III – *Etude clinique*

III.1 – *But de l'étude*

Cette étude avait pour but de comparer chez l'homme la biodisponibilité de l'oxalate de naftidrofuryl et du citrate de naftidrofuryl, biodisponibilité qui a été appréciée par la mesure des taux plasmatiques et par la détermination des compliances veineuses.

III.2 – *Matériel et méthode*

III.2.1 – Sujets: l'essai a été réalisé sur 12 sujets (9 sujets de sexe féminin et 3 sujets de sexe masculin), l'âge moyen étant de 52,5 années.

III.2.2 – Description de l'étude: chaque sujet a reçu par voie orale au moins à 8 jours d'intervalle, soit 300 mg d'oxalate de naftidrofuryl, soit 300 mg de citrate de naftidrofuryl. 10 h avant la prise médicamenteuse et jusqu'à 4 h après celle-ci, les sujets n'ont pas absorbé d'aliments. Les échantillons de sang veineux ont été recueillis sur héparine-fluorure; les taux plasmatiques de naftidrofuryl ont été mesurés selon la technique de Fontaine *et al.* (Bull. Chim. Thér. 1969, *1*, 44-49) aux temps 0 (avant administration), 0,5; 1; 1,5; 2; 3; 4; 6; 8 et 12 h après l'administration de l'un ou l'autre sel de naftidrofuryl. En outre, chaque demi-heure suivant l'administration des produits testés a été mesurée la vasodilatation obtenue par l'un ou l'autre sel de naftidrofuryl après vasoconstriction préalable par administration répétée de dihydroergotamine suivant la technique de Thébault *et al.* (Comm. World Conférence on Clinical Pharmacology and Therapeutics, Londres, 1980) dont la description succincte est donnée ci-après:

Le procédé repose sur la détermination optique du calibre d'une veine superficielle de la main, à une pression congestive standard. Le point culminant d'une veine appropriée du dos de la main est marqué d'une croix à l'encre de Chine. Le sujet, qui se trouve dans une pièce maintenue à température constante – demeure étendu, son membre supérieur, qui repose sur une attelle rigide, faisant un angle de 30° par rapport à l'horizontale, de manière que les veines superficielles soient entièrement collabées. Un microscope binoculaire dont l'axe optique se trouve exactement situé à la perpendiculaire de la surface cutanée, est mis au point sur la croix formant repère. Une à deux minutes après la mise en place d'un garrot, on fait la mise au point et on considère la veine superficielle appropriée comme étant totalement vasodilatée; on donne alors arbitrairement la valeur 100 au diamètre de la veine considérée. On lui administre alors (temps 0) une dose de charge de 2 mg de dihydroergotamine

(DHE) et, 90 min après (soit temps 1,5), on lui administre, toutes les 30 min, pendant toute la durée de l'essai (6 h) une dose de 0,5 mg de DHE. Ces doses ont été choisies lors d'essais préalables qui ont permis d'obtenir une vasoconstriction permanente sans que le sujet présente de manifestations secondaires. Au temps 1,5, on administre au sujet, en une seule dose et en une seule fois, soit 300 mg d'oxalate de naftidrofuryl, soit 300 mg de citrate de naftidrofuryl. Le diamètre de la veine superficielle est mesuré toutes les demi-heures, pendant 6 h.

III.3 – *Résultats*

Les résultats moyens obtenus sont consignés dans les tableaux suivants.

Le Tableau I concerne les taux plasmatiques et les aires sous les courbes et le Tableau II le pourcentage de vasodilatation résiduelle.

*Tableau I*

| Produit administré \ T (h) | 0,5 | 1 | 1,5 | 2 | 3 | 4 | 6 | 8 | 12 | aires s/les courbes |
|---|---|---|---|---|---|---|---|---|---|---|
| Oxalate de Naftidrofuryl | 1,22 | 1,11 | 0,69 | 0,58 | 0,24 | 0,13 | 0,05 | 0,02 | 0,01 | 2,56 |
| Citrate de Naftidrofuryl | 2,90 | 2,47 | 1,68 | 1,05 | 0,60 | 0,42 | 0,24 | 0,08 | 0,02 | 6,30 |

*Tableau II*

Vasodilatation observée après administration d'oxalate de naftidrofuryl ou de citrate de naftidrofuryl

| Produit administré \ T (h) | 0 | 0,5 | 1 | 1,5 | 2 | 2,5 | 3 | 3,5 | 4 | 4,5 | 5 | 5,5 | 6 | aires s/les courbes |
|---|---|---|---|---|---|---|---|---|---|---|---|---|---|---|
| Oxalate de Naftidrofuryl | 0 | 69 | 87 | 61 | 35 | 22 | 13 | 4 | 0 | 0 | 0 | 0 | 0 | 145,5 |
| Citrate de Naftidrofuryl | 0 | 28 | 35 | 48 | 65 | 83 | 91 | 78 | 56 | 39 | 22 | 4 | 0 | 260,5 |

L'examen des résultats permet de formuler les observations suivantes:

III.3.1 – Taux plasmatiques: la simple lecture du Tableau I montre que la biodisponibilité du citrate de naftidrofuryl est non seulement nettement plus importante que celle de l'oxalate de naftidrofuryl, mais qu'elle s'étale dans le temps puisque, quel que soit le temps observé, le taux de naftidrofuryl est toujours en faveur du citrate. Ce qui précède est confirmé par l'aire sous la courbe qui subit une augmentation de 146%, toujours en faveur du citrate.

III.3.2 – Vasodilatation (tableau II): là encore, il est constaté une biodisponibilité nettement supérieure et toujours en faveur du citrate de naftidrofuryl mais en outre et assez curieusement un effet nettement retardé dans le temps: alors que la vasodilatation maximale est observée aux temps 0,15-1 h pour l'oxalate de naftidrofuryl elle passe aux temps 2,5-3 h pour le citrate de naftidrofuryl. En outre, les aires sous les courbes de vasodilatation, exprimées en % de vasodilatation × heures sont respectivement en moyenne de 145,5 pour l'oxalate de naftidrofuryl et de 260,5 pour le citrate de naftidrofuryl.

Il découle de l'ensemble des résultats expérimentaux décrits ci-dessus que:

1) – *Sur le plan toxicologique*

Le citrate de naftidrofuryl est nettement mieux toléré que l'oxalate de naftidrofuryl, les $DL_{50}$ observées étant en faveur, dans des proportions importantes, du citrate de naftidrofuryl.

2) – *Sur le plan pharmacologique*

La tolérance et l'activité cardiaque et respiratoire sont également en faveur du citrate de naftidrofuryl.

3) – *Sur le plan clinique*

La biodisponibilité, qui est en faveur du citrate de naftidrofuryl, est confirmée par l'aire sous la courbe; il en va de même pour le test de Thébault *et al.*, mais, curieusement, la vasodilatation maximale a mis en évidence pour le citrate de naftidrofuryl, mais non pour l'oxalate de naftidrofuryl, un effet nettement retardé dans le temps.

Les caractéristiques toxicologiques, pharmacologiques et cliniques du citrate de naftidrofuryl qui se traduisent par rapport à l'oxalate de naftidrofuryl par une meilleure tolérance, une meilleure activité,

une meilleure biodisponibilité et un effet retardé dans le temps, sont en faveur d'une meilleure adéquation du citrate de naftidrofuryl à sa fonction thérapeutique comme par exemple, dans le traitement de l'artérite des membres, de la maladie de Raynaud et de l'insuffisance circulatoire cérébrale.

Le médicament de l'invention peut être présenté, pour l'administration orale, sous forme de comprimés, comprimés dragéifiés, ou gélules. Il peut aussi être présenté pour l'administration parentérale, sous forme de soluté injectable par les voies intramusculaires ou intraveineuses ou en perfusion veineuse.

Chaque dose unitaire contient avantageusement de 0,010 g à 0,500 g de principe actif, les doses administrables journellement pouvant varier de 0,040 g à 1,00 g de principe actif selon l'âge du patient et la gravité de l'affection traitée.

On donnera ci-après, à titre d'exemples non limitatifs, quelques formulations pharmaceutiques du médicament de l'invention.

1) Comprimés
Citrate de naftidrofuryl 0,200 g
Excipient: amidon de maïs, acide alginique, polyvidone, stéarate de magnésium, talc.

2) Comprimés dragéifiés
Citrate de naftidrofuryl 0,150 g
Excipient: talc, polyvinylpyrrolidone, stéarate de magnésium, gomme arabique, glucose, cire blanche, cire de carnauba, lactose, oxyde de titane.

3) Gélules
Citrate de naftidrofuryl 0,050 g
Excipient: talc, stéarate de magnésium

4) Soluté injectable
Citrate de naftidrofuryl 0,040 g
Excipient: solvant isotonique q.s.p. 5 ml

**Revendications** pour les Etats contractants, BE, CH, DE, FR, GB, IT, LI, LU, NL, SE

1. Le citrate de naftidrofuryl ((naphtyl-1)-3 tétrahydrofurfuryl-2 propionate de diéthylamino-2 éthyl).

2. Médicament ayant notamment des propriétés d'activateur métabolique et circulatoire et des propriétés vasoactives dans le traitement de l'artérite des membres, de la maladie de Raynaud et de l'insuffisance circulatoire cérébrale, caractérisé en ce qu'il contient à titre de principe actif du citrate de naftidrofuryl.

3. Médicament suivant la revendication 2, caractérisé en ce qu'il est présenté sous forme de comprimés, comprimés dragéifiés, gélules pour l'administration orale et de soluté injectable pour l'administration par voie parentérale, le principe actif étant associé aux véhicules appropriés.

4. Médicament selon l'une des revendications 2 ou 3, caractérisé en ce qu'il est présenté sous forme de doses unitaires contenant de 0,010 g à 0,500 g de principe actif.

**Revendications** pour l'Etat contractant: AT

1. Procédé de préparation du citrate de naftidrofuryl ((naphtyl-1)-3 tétrahydrofurfuryl-2 propionate de diéthylamino-2 éthyle), caractérisé en ce qu'on fait réagir le naftidrofuryl avec l'acide citrique.

2. Procédé selon la revendication 1, caractérisé en ce que l'acide citrique est utilisé sous forme monohydratée.

**Patentansprüche** für die Vertragsstaaten: BE, CH, DE, FR, GB, IT, LI, LU, NL, SE

1. Naphthydrofurylcitrat (3-(1-Naphthyl)-2-tetrahydrofurfurylpropionat von 2-Diethylaminoethyl).

2. Medikament mit insbesondere den Stoffwechsel und den Kreislauf aktivierenden Eigenschaften und vasoaktiven Eigenschaften bei der Behandlung der Arthritis der Gliedmassen, der Raynaudschen Krankeit und der zerebralen Kreislaufinsuffizienz, dadurch gekennzeichnet, dass es als Wirkstoff Naphthydrofurylcitrat enthält.

3. Medikament nach Anspruch 2, dadurch gekennzeichnet, dass es in Form von Tabletten, dragierten Tabletten, Kapseln für die orale Verabreichung und Injektionslösung für die parenterale Verabreichung vorliegt, wobei der Wirkstoff mit geeigneten Trägern vereinigt ist.

4. Medikament nach Anspruch 2 oder 3, dadurch gekennzeichnet, dass es in Form von Einheitsdosen, die 0,010 g bis 0,500 g Wirkstoff enthalten, vorliegt.

**Patentansprüche** für den Vertragsstaat: AT

1. Verfahren zur Herstellung von Naphthydrofurylcitrat (3-(1-Naphthyl-2-tetrahydrofurfurylpropionat von 2-Diethylaminoethyl), dadurch gekennzeichnet, dass man Naphthydrofuryl mit Citronensäure umsetzt.

2. Verfahren nach Anspruch 1, dadurch gekennzeichnet, dass die Citronensäure in Form des Monohydrats verwendet wird.

**Claims** for the Contracting States: BE, CH, DE, FR, GB, IT, LI, LU, NL, SE

1. Naftidrofuryl(-(2-diethylaminoethyl 3-(1-naphtyl)-2-tetrahydrofurfuryl-propionate) citrate.

2. Medicament having properties of metabolic and circulatory promoter and vaso-active properties in the treatment of arteritis of the limbs, of the Raynaud's disease and of cerebral circulatory insufficiency, comprising as active ingredient naftidrofuryl citrate.

3. Medicament according to Claim 2, characterized in that it is in the form of tablets, dragees, capsules for oral administration, and in the form of injectable solutions for parenteral administration, the active ingredient being together with suitable vehicles.

4. Medicament according to Claim 2 or 3, characterized in that it is in the form of dosage units containing 0,010 g to 0,500 g of active ingredient.

**Claims** for the Contracting State: AT

1. Process for preparing naftidrofuryl-(2-diethylaminoethyl 3-(1-naphtyl)-2-tetrahydrofurfurylpropionate) citrate, wherein naftidrofuryl is reacted with citric acid.

2. Process according to Claim 1, wherein citric acid is used in the monohydrate form.